# EUROPÄISCHE PATENTSCHRIFT

(11) **EP 1 753 618 B1**
(45) Veröffentlichungstag und Bekanntmachung des Hinweises auf die Patenterteilung: **08.07.2009**
(21) Anmeldenummer: 05747787.9
(22) Anmeldetag: 21.05.2005
(51) Int. Cl.: B32B 37/14, B32B 37/28, B32B 38/00

(54) **VERFAHREN ZUR HERSTELLUNG VON ELASTISCHEM BAHNMATERIAL**
METHOD FOR PRODUCING AN ELASTIC WEB MATERIAL
PROCEDE POUR PRODUIRE UNE BANDE DE MATIERE ELASTIQUE

(30) Priorität: 25.05.2004 DE 102004026070
(43) Veröffentlichungstag der Anmeldung: 21.02.2007
(73) Patentinhaber: Fenske, Sandra, 64683 Einhausen (DE)
(72) Erfinder: Fenske, Sandra, 64683 Einhausen (DE)
(74) Vertreter: Helber, Friedrich
(86) Internationale Anmeldenummer: PCT/EP2005/005527
(87) Internationale Veröffentlichungsnummer: WO 2005/115754

(56) Entgegenhaltungen:
- EP-A- 0 803 602
- WO-A-03/041627
- PATENT ABSTRACTS OF JAPAN Bd. 2002, Nr. 11, 6. November 2002 (2002-11-06) & JP 2002 192641 A (NIPPON PETROCHEM CO LTD; POLYMER PROCESSING RES INST), 10. Juli 2002 (2002-07-10)

## Beschreibung

Die Erfindung betrifft ein Verfahren zur kontinuierlichen Herstellung eines biegeweichen luftdurchlässigen Bahnmaterials mit zwei äußeren Schichten, von denen wenigstens eine von einem textilen Material gebildet wird, zwischen denen eine unter Vorspannung stehende elastische Zwischenlage eingebracht ist, durch welche das Bahnmaterial unter Entspannung der Zwischenlage verkürzt und durch Ausübung von Zugbeanspruchung wieder elastisch verlängerbar ist, bei dem zunächst das Vorderende der ersten äußeren Schicht auf den Endabschnitt eines langgestreckten stab- oder rohrartigen Formkerns aufgebracht und unter gleichzeitiger Erteilung einer Vorschubbewegung auf dem Formkern in eine schlauchartig geschlossene Form um den Formkern herumgefaltet und dann während der Vorschubbewegung auf dem Formkern auf die frei liegende Oberseite der ersten äußeren Schicht Haftkleber aufgebracht wird, worauf die schlauchartige erste Schicht dann im Zuge der weiteren Vorschubbewegung auf dem Formkern durch eine Zuführeinrichtungen für eine Gruppe von unter elastischer Vorspannung stehenden elastischen Fäden oder Strängen hindurch geführt wird, wobei die elastischen Fäden oder Stränge in der Zuführeinrichtung auf einem den langgestreckten Formkern ringförmig umgebenden Träger in in Umfangsrichtung voneinander beabstandet angeordneten Einzelführungen geführt unter Vorspannung auf die erste äußere Schicht aufgebracht und durch den Haftkleber mit dieser verbunden werden, wobei der ringförmigen Träger der Einzelführungen während der Vorschubbewegung der schlauchförmigen ersten Schicht auf dem Formkern um die Längsachse des Formkerns drehangetrieben wird, so dass die Fäden oder Stränge aufgrund der überlagerten Vorschubbewegung der ersten Schicht und der Drehung des Trägers schräg verlaufend und im Wesentlichen parallel zueinander ausgerichteten auf der schlauchförmigen ersten Schicht aufgebracht werden und die schlauchartige erste Schicht jeweils nach Art einer Schlaucharmierung umgeben, und auf die mit den aufgebrachten Gruppen von elastischen Fäden oder Strängen belegte schlauchartige erste Materialschicht dann die zweite Materialschicht aufgebracht und während der weiteren Vorschubbewegung auf dem Formkern schlauchförmig um diese herumgefaltet und durch Haftverbindung mit der ersten Materialschicht und den aufgebrachten elastisch Fäden bzw. Strängen verbunden wird, worauf das so erzeugte schlauchförmige Bahnmaterial in Längsrichtung aufgeschnitten und zu dem im Wesentlichen ebenflächigen Bahnmaterial aufgefaltet wird.

Textile Materialien, die aus einem in ungespanntem Ausgangszustand in zumindest einer Koordinatenrichtung elastisch ausziehbar sind, werden insbesondere im Zusammenhang mit Unterbekleidung und Miederwaren eingesetzt, welche aufgrund der Materialelastizität einen hinreichend festen großflächigen Sitz von Bekleidungsstücken gewährleistet, an oder in welchen das Material verarbeitet ist, um Nachteile von gesonderte angenähten oder eingezogenen, bei hinreichend festem Sitz streifenförmige Druckstellen im Körper des Benutzers erzeugenden Gummibändern, z.B. in der Taille oder im Beinabschluss von Unterhosen oder Slips zu vermeiden. Durch Verwendung solcher flächen-elastischen Materialien ist eine Verarbeitung möglich, bei welcher eine großflächige Anlage des Bekleidungsstücks am Körper mit relativ geringem Flächendruck bei gleichzeitig gutem Sitz erhalten wird. Textilmaterialien, die aus oder unter Mitverwendung von elastomeren Fasern in Form eines gewirkten Gewebes hergestellt werden, erlauben die Herstellung elastischer Unterwäsche, z.B. von Miederhöschen, Stützstrümpfen etc. Diese Materialien sind allerdings teuer in der Herstellung und deshalb nicht für die Verarbeitung in preiswerter Unterbekleidung geeignet, welche nur einmal verwendet werden soll, z.B. für Inkontinenz-Unterhosen oder auch Windelhöschen für Babys. Gerade bei solchen Anwendungen ist aber ein preisgünstiges Material erforderlich, welches bei sicherem Halt gegen versehentliches Abstreifen des Wäschestücks einen dichten Abschluss insbesondere im Beinbereich gewährleistet, um sicherzustellen, dass keine flüssigen Körperausscheidungen aus der in der Regel vorgesehenen Saugeinlage austreten können.

Aus der WO 03/041627A ist ein Verfahren der eingangs erwähnten Art bekannt, bei welchem ein bzw. eine Gruppe von elastischen Fäden oder Strängen aus einem nicht- oder latent-elastischen Material um ein auf einem langgestreckten Kern zu einem Schlauch geformten Textilmaterial spiralförmig aufgebracht und anschließend fixiert wird. Das zum Schlauch geformte Textilmaterial wird kontinuierlich auf dem Kern vorgeschoben und gleichzeitig der Faden bzw. die Gruppe von Fäden mit einer Vorspannung über ein sich um die Achse des Schlauchmaterials drehende Zuführeinrichtung auf der zuvor mit einem Haftkleber versehenen Oberfläche des Schlauchmaterials aufgebracht, welches dann aufgefaltet wird. In einer abschließenden, z. B. von einem "ring-rolling process"gebildeten Aktivierungsbehandlung wird in dem Laminat die angestrebte Elastizität erzeugt. Da bei diesem Verfahren in aufgefaltetem Zustand des Materials die elastischen Fäden oder Strängeparallel gleichsinnig schräg im Material verlaufen, ist das Material in aufgefaltetem Zustand im Wesentlichen nur in Richtung des Längsverlaufs der Fäden oder Stränge elastisch dehnbar.

Der Erfindung liegt demgegenüber die Aufgabe zugrunde, ein Herstellungsverfahren zu schaffen, welches die kontinuierliche Herstellung von flächenelastischem textilen Bahnmaterial mit hoher Produktionskapazität und - hieraus resultierend - preisgünstig ermöglicht, wobei das Endprodukt flächenelastische Eigenschaft aufweist.

Ausgehend von einem Verfahren der eingangs erwähnten Art wird diese Aufgabe erfindungsgemäß dadurch gelöst, dass der die Gruppe von elastischen Fäden oder Strängen auf die erste Mateialschicht aufgebringenden Zuführvorrichtung in Vorschubrichtung nachgeschaltete eine weitere in entgegengesetztem Drehsinn angetriebene zweite Zuführvorrichtung vorgesehen wird, mittels welcher jeweils eine weitere Gruppe von im Wesentlichen paralell zueinander ausgerichteten gegensinnig schräg zu der ersten Gruppe verlaufenden elastischen Fäden oder Strängen auf der schlauchartigen ersten Materilschicht aufgebracht und haftend mit dieser verbunden wird, und dass die elastischen Fäden oder Stränge beider Gruppen während der Aufbringung auf der schlauchförmigen ersten Materialschicht in gegenüber ihrem ungespannten Zustand durch eine der gewünschten elastischen Dehnung des fertigen Bahnmaterials entsprechende Vorspannung auf ein vergrößerte Länge gereckt mit der schlauchförmigen ersten Materialschicht verbunden und dann die zweite Materialschicht in noch gerecktem Zustand der elastischen Fäden oder Stränge auf die erste Materialschicht aufgebracht und haftend mit ihr verbunden wird. Durch die Aufbringung der elastischen Fäden bzw. Stränge in der zweiten Zuführvorrichtung in entgegengesetztem Drehsinn zu den in der ersten Zuführvorrichtung aufgebrachten Fäden bzw. Stränge wird erreicht, dass die Umfangskräfte im resultierenden Schlauch weitgehend neutralisiert werden und der Schlauch somit unabhängig von der Höhe der Vorspannung - frei von jeglicher Verdrillung - axial aufgetrennt werden kann und damit ohne weitere Aktivierung eventueller latenter elastischer Eigenschaften in einen in Produktions - Querrichtung endelastischen Zustand gebracht wird.

Dabei wird vorzugsweise so verfahren, dass der Haftkleber vor, während und/oder nach dem Aufbringen der elastischen Fäden oder Stränge auf der freien Oberfläche der ersten Materialschicht auf die elastischen Fäden oder Stränge allein oder auf die elastischen Fäden oder Stränge und die erste Materialschicht während deren Vorschubbewegung auf dem Formkern aufgebracht wird. Als erste Materialschicht wird dabei die Schicht aus textilem Material verwendet.

Als zweite Materialschicht kann alternativ ebenfalls eine Schicht aus textilem Material oder eine Folie verwendet werden.

Im Falle der Verwendung einer Folie empfiehlt es sich, eine Folie aus einem gas- oder wasserdampfdurchlässigen, gegen Flüssigkeiten jedoch undurchlässigen Folienmaterial zu verwenden.

Dabei kann auch so verfahren werden, dass auf der mit aufgebrachten Gruppen von elastischen Fäden oder Strängen belegten ersten Materialschicht als zweite Materialschicht eine Lage eines in fließfähigem Zustand befindlichen Kunststoffs aufgebracht, z.B. aufgestrichen oder aufgesprüht wird, der nachfolgend in einen biegeweichen folien-oder vliesartigen Zustand übergeführt wird.

Als Kunststoff wird dann mit Vorteil ein durch Erwärmung in fließfähigen Zustand gebrachtes thermoplastisches Kunststoffmaterial verwendet, welches nach der Aufbringung auf der mit aufgebrachten Gruppen von elastischen Fäden oder Strängen belegten ersten Materialschicht durch Abkühlung in den folien- oder vliesartigen Zustand übergeführt wird. Alternativ kann als Kunststoff auch ein durch Verdunstung oder Verdampfung eines Lösungsmittelanteils verfestigbares oder ein durch Vermischen von wenigstens zwei flüssigen Komponenten durch Vernetzung verfestigbares Kunststoffmaterial verwendet werden.

In vorteilhafter Weiterbildung der Erfindung kann zur Durchführung des Verfahrens ein mit wenigstens zwei in Umfangsrichtung zueinander versetzten, in Längsrichtung verlaufenden schlitz- oder rinnenförmigen Vertiefungen versehener Formkern verwendet werden, wobei dann beim Aufbringen der ersten Materialschicht auf den Formkern über den schlitz- bzw. rinnenförmigen Vertiefungen aufgebrachte streifenförmige Bereiche der Materialschicht in die Vertiefung eingeformt und während der Vorschubbewegung in den Vertiefungen gehalten werden. Beim Aufbringen der Gruppen von elastischen Fäden oder Strängen werden dann die in die Vertiefungen eingeformten Materialbereiche nicht mit solchen elastischen Fäden oder Strängen belegt und haftend miteinander verbunden, d.h. es bilden sich im späteren aufgeschnittenen Bahnmaterial in Produktionsrichtung verlaufende parallele streifenförmige Materialbereiche, die abwechselnd mit elastischen Fäden oder Strängen belegt bzw. frei von solchen elastischen Fäden oder Strängen sind. Das so erzeugte Bahnmaterial weist also nur in den mit den elastischen Fäden bzw. Strängen belegten streifenförmigen Bereichen Elastizität auf, während die dazwischen liegenden streifenförmigen Bereiche keine - oder allenfalls im Maße einer eventuellen Elastizität des Grundmaterials - elastische Eigenschaften aufweist.

In Weiterbildung der Erfindung kann vor oder während der Aufbringung der zweiten Materialschicht auf der mit Gruppe von elastischen Fäden oder Strängen versehenen ersten Materialschicht zusätzlich ein pulver-, flocken- oder faserförmiger Füll- oder Funktionsstoff aufgebracht werden, welcher z.B. hygroskopische, d.h. Flüssigkeiten aufnehmende und zurückhaltende Eigenschaften hat. Auch die Erhöhung des Tragekomforts des Materials durch polsternde Eigenschaften des Füll- oder Funktionsstoffs kann auf diese Weise verwirklicht werden.

Die Erfindung ist in der folgenden Beschreibung in Verbindung mit den Zeichnungsfiguren näher erläutert, welche schematisch die Durchführung des erfindungsgemäßen Verfahrens bei der Herstellung von flächenelastischem Bahnmaterial veranschaulichen. Es zeigt bzw. zeigen:
- Fig. 1: eine Seitenansicht von wesentlichen Funktionselementen einer zur Durchführung des erfindungsgemäßen Verfahrens verwendbaren Vorrichtung in stark schematisierter Darstellung;
- Fig. 2: eine perspektivische Ansicht der in Fig. 1 gezeigten Vorrichtung;
- Fig. 3a: die Anordnung von Einzelführungen in einer der in der Vorrichtung gemäß den Figuren 1 und 2 zur Aufbringung von Gruppen von vorgespannten elastischen Fäden oder Strängen vorgesehenen Zuführeinrichtungen;
- Fig. 3b: das bei Verwendung von zwei gegensinnig angetriebenen Zuführvorrichtungen gemäß Figur 3a erhaltene Flächenmuster der elastischen Fäden oder Stränge in einem in der erfindungsgemäßen Weise hergestellten Bahnmaterial;
- Fig. 4a: eine abweichende schematische Anordnung von Einzelführungen in einer der in der Vorrichtung gemäß den Figuren 1 und 2 zur Aufbringung von Gruppen von vorgespannten elastischen Fäden oder Strängen vorgesehenen Zuführeinrichtungen;
- Fig.4b: das bei Verwendung von zwei gegensinnig angetriebenen Zuführvorrichtungen gemäß Figur 4a erhaltene Flächenmuster der elastischen Fäden oder Stränge in einem in der erfindungsgemäßen Weise hergestellten Bahnmaterial;
- Fig. 5a: einen rechtwinklig zur Längserstreckung eines Formkerns verlaufende Schnittansicht durch einen abgewandelten Formkern im Bereich einer Zuführvorrichtung für die elastischen Fäden oder Stränge; und
- Fig. 5b: die Darstellung des bei Verwendung des in Figur 5a gezeigten Formkerns bei der Herstellung des Bahnmaterials erzeugten Flächenmusters der elastischen Fäden oder Stränge.

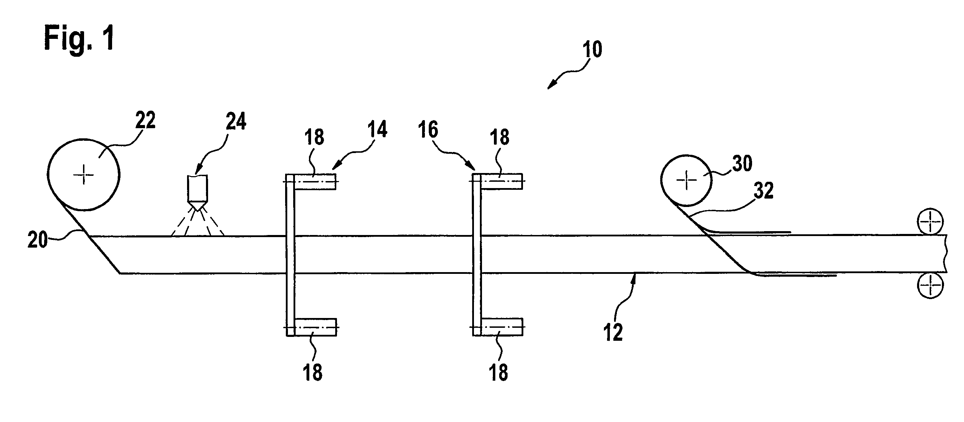

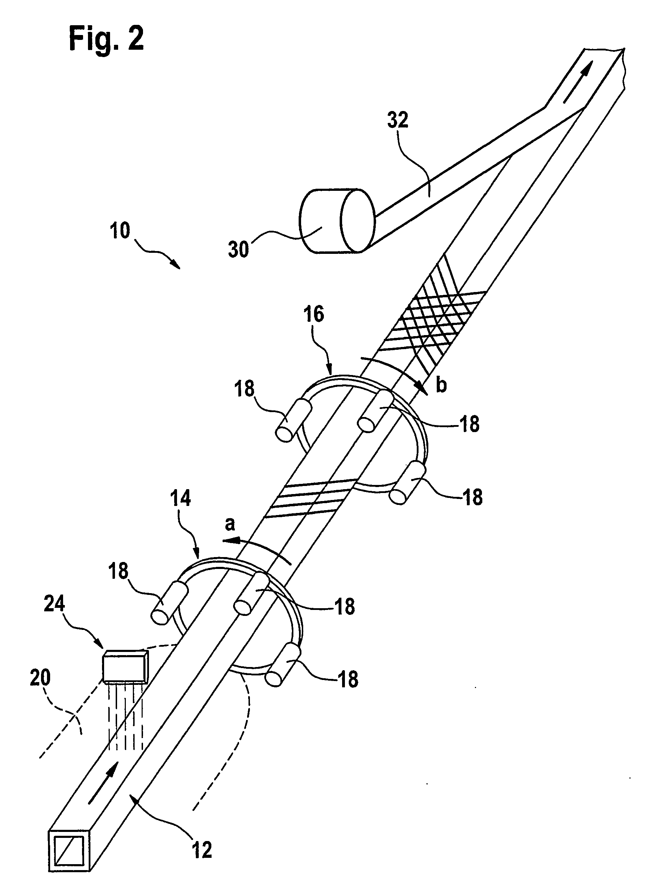

Die in den Figuren 1 und 2 stark schematisiert und vereinfacht dargestellte Vorrichtung 10 zur Durchführung des erfindungsgemäßen Verfahrens weist einen langgestreckten, im dargestellten Fall im Querschnitt quadratischen Formkern 12 auf, welcher von zwei in Längsrichtung voneinander beabstandeten Zuführeinrichtungen 14 und 16 konzentrisch umgeben wird. Die Zuführeinrichtungen 14 und 16 sind in der durch die Pfeile a bzw. b veranschaulichten Drehrichtung gegensinnig drehantreibbar, wobei die genaue konstruktive Ausgestaltung und Lagerung der den Formkern umgebenden Zuführeinrichtungen und deren Antriebe aus Gründen der Übersichtlichkeit nicht dargestellt sind.

In den Zuführeinrichtungen 14 und 16 sind jeweils in Umfangsrichtung auf einem ringförmigen Träger verteilt voneinander beabstandet angeordnete Einzelführungen 18 für elastische Fäden oder Stränge angeordnet, über welche jeweils ein solcher elastischer Faden oder Strang unter Vorspannung oder -dehnung in Richtung zum Formkern 12 geführt werden kann.

Vor dem in Figur 1 links und in Figur 2 unten befindlichen Ende des Formkerns 12 ist eine ebenflächige textile Materialbahn 20 zu einer Rolle 22 aufgewickelt vorgesehen, wobei die Rolle 22 der Materialbahn auf einer Halterungsachse drehbar gelagert ist, so dass das Material 20 von der Rolle abgewickelt und auf den Formkern 12 aufgebracht und dann aus einer Auftragevorrichtung 24 mit einem fließfähigen Haftkleber besprüht werden kann. Die erste vliesartige Materialbahn wird dann in eine schlauchartig geschlossene Form um den Formkern herumgefaltet und in dieser schlauchartig geschlossenen oder teilgeschlossenen Form kontinuierlich auf dem Formkern 12 - in Figur 1 nach rechts und Figur 2 schräg in Aufwärtsrichtung - weitergeführt, wobei das nunmehr schlauchartig und mit noch unabgebundenem bzw. nicht verfestigten Haftkleber besprühte vliesartige Material aufeinanderfolgend durch die Zuführeinrichtungen 14 und 16 hindurch tritt.

In den Zuführeinrichtungen wird von deren sich mit der jeweiligen Zuführeinrichtung drehenden Einzelführungen 18 jeweils ein elastischer Faden bzw.

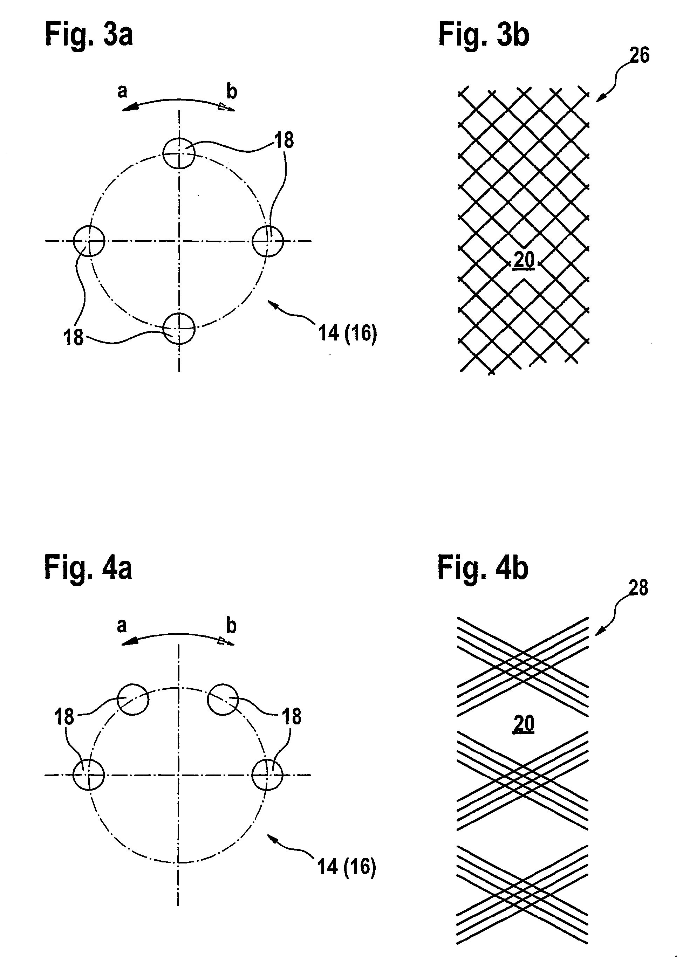

Strang unter Vorspannung auf die Materialbahn 20 aufgebracht und dort durch den Haftkleber 24 - nach dessen Abbinden - fixiert. Durch die Überlagerung der Drehbewegung der Einzelführungen 18 in den gegensinnig angetriebenen Zuführeinrichtungen 14, 16 und die Vorschubbewegung des schlauchartig geschlossenen Materialbahn werden die unter Vorspannung stehenden Fäden oder Stränge in einem Flächenmuster auf dem ersten vliesartigen Material fixiert, welches von zwei gegensinnig schräg verlaufende Gruppen von in jeder Gruppe im Wesentlichen parallel zueinander ausgerichteten Fäden oder Stränge gebildet wird. Dieses Flächenmuster, welches z.B. mit der in Figur 3a schematisch veranschaulichten Anordnung von vier Einzelführungen 18 in gleichmäßigen Winkelabständen von jeweils 90° auf der Zuführvorrichtung 14 erzeugt wird, ist in Figur 3b veranschaulicht. Es ist erkennbar, dass das dort dargestellte Flächenmuster 26 von gegensinnig schräg verlaufenden und sich überkreuzenden Fäden gebildet wird.

Wenn die Einzelführungen 18 in der in Figur 4a veranschaulichten Weise nur über einen Teil des gesamten Umfangs der Zuführeinrichtung 14 (und 16) angeordnet sind, entsteht ein Flächenmuster 28 von vorgespannten elastischen Fäden oder Strängen auf dem Material 20, wie es in Figur 4b veranschaulicht ist.

Nach Durchtritt der schlauchförmigen Materialbahn 20 durch die zweite Zuführeinrichtung 16 hindurch wird von einer zweiten Vorratsrolle 30 eine weitere Lage eines auch hier wieder als Vlies angenommenen textilen Materials 32 abgewickelt, auf die mit den elastischen Fäden bzw. Strängen belegte, formkernabgewandte Außenseite der ersten Materialbahn 20 aufgebracht und ebenfalls in eine schlauchförmig geschlossene Form herumgefaltet und durch den noch nicht abgebundenen Haftkleber auf der ersten Materialbahn 20 fixiert. Dadurch entsteht insgesamt ein zweilagiger Schlauch, zwischen dem die elastischen Fäden bzw. Stränge in dem erzeugten Flächenmuster nach Art einer Schlaucharmierung eingeschlossen sind.

Sobald der Haftkleber dann abgebunden hat und die beiden äußeren Schichten mit den dazwischen liegenden elastischen Fäden haftend verbindet, wird der so gebildete Schlauch beim weiteren Produktionsvorschub aufgeschnitten und dann zu einem ebenflächigen Bahnmaterial aufgefaltet, welches dann wieder als Endprodukt auf eine Rolle aufrollbar ist.

Nach dem Aufschneiden des Schlauchs wird das so entstandene ebenflächige Bahnmaterial dann durch die Vorspannung der Armierung von elastischen Fäden oder Strängen elastisch zusammengezogen und verkleinert so seine Fläche, wobei also ein flächenelastisches, d.h. unter Erhöhung der Spannung wieder elastisch auseinander ziehbares Bahnmaterial entsteht, aus dem dann die Zuschnitte für die Weiterverarbeitung zu Bekleidungsstücken bzw. Miederwaren hergestellt werden können.

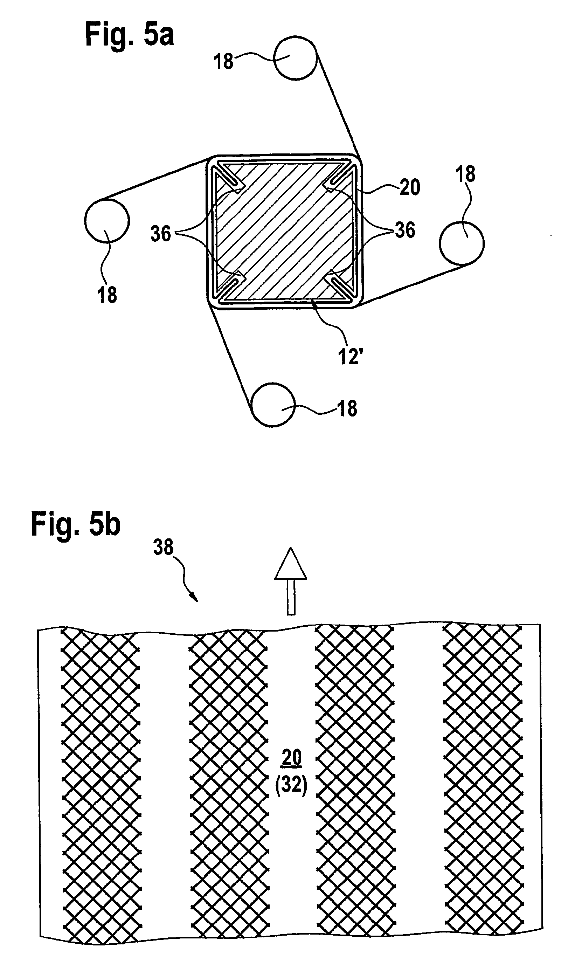

In Figur 5a ist ein abgewandelter Formkern 12' der Vorrichtung 10 in Zuordnung zu den Einzelführungen 18 einer Zuführeinrichtung 14 oder 16 im Querschnitt gezeigt. Der Formkern 12' unterscheidet sich dadurch von dem in Verbindung mit den Figuren 1 und 2 beschriebenen Formkern 12, dass jeweils im Bereich der Längskanten des Formkerns längsverlaufende Schlitze oder rinnenförmige Vertiefungen 36 eingearbeitet sind, in welche während des Herstellungsvorgangs des Bahnmaterials die jeweilige Materialbahn 20 und 32 durch ein entsprechendes spatelartiges Führungselement eingefaltet werden kann, wie dies in Figur 5a in Bezug auf die Materialbahn 20 veranschaulicht ist. Es ist ersichtlich, dass bei Verwendung dieses Formkerns und Einfalten der Materialbahnen 20 und 32 in die längsverlaufenden Schlitze oder rinnenförmigen Vertiefungen 36 die eingefalteten Bereiche der Materialbahnen 20 bzw. 32 nicht mit dem Haftkleber besprüht und auch nicht mit den vorgespannten elastischen Fäden oder Strängen belegt werden.

Nach dem Aufschneiden des im Übrigen in der bereits beschriebenen Weise hergestellten erfindungsgemäßen Bahnmaterials entsteht dann ein Bahnmaterial mit dem in Figur 5b veranschaulichten Flächenmuster 38, bei dem jeweils in Produktionsrichtung verlaufende parallele streifenförmige Bereiche entstehen, die abwechselnd durch elastische Fäden oder Stränge flächenelastisch bzw. nicht oder nur im Rahmen der Elastizität der Fasern des textilen Bahnmaterials elastisch sind.

Es ist ersichtlich, dass im Rahmen der Erfindung Abwandlungen und Weiterbildungen des vorstehend beschriebenen erfindungsgemäßen Verfahrens verwirklichbar sind. Solche Abwandlungen können beispielsweise darin bestehen, dass die gegensinnig angetriebenen Zuführeinrichtungen 14 und 16 mit unterschiedlicher Drehgeschwindigkeit angetrieben oder von ihrer, den Formkern rechtwinklig umgebenden Lage aus in eine schräge Lage zur Längsmittelachse des Formkerns versetzt angeordnet werden. Hierdurch werden entsprechende Änderungen des Flächenmusters der elastischen Fäden bzw. Stränge erhalten. Anstelle der Verwendung von zwei textilen Materialbahnen kann das Verfahren auch so durchgeführt werden, dass nur die erste Materialbahn 20 ein textiles Material, beispielsweise ein Vlies ist, während die zweite Materialbahn 32 von einer Folienbahn gebildet wird, die entweder in Form einer Folie aufgebracht wird, oder aber die durch Erwärmung oder Beifügung von flüchtigem Lösungsmittel fliesfähigen Zustand gebracht und aufgespritzt wird und anschließend durch Abkühlen oder Abdampfen des Lösungsmittels zur Folienschicht erstarrt.

Weiterhin kann es sinnvoll und vorteilhaft sein, wenn vor oder während der Aufbringung der zweiten Materialbahn 32 auf der mit den elastischen Fäden oder Strängen versehenen ersten Materialbahn ein pulver-, flocken- oder faserförmiger Füll- oder Funktionsstoff aufgebracht wird, welcher beispielsweise den Tragekomfort durch Polsterwirkung erhöht oder auch die Funktion eines Saugkissens für Flüssigkeiten haben kann.

## Patentansprüche

1. Verfahren zur kontinuierlichen Herstellung eines biegeweichen luftdurchlässigen Bahnmaterials mit zwei äußeren Schichten (20; 32), von denen wenigstens eine von einem textilen Material gebildet wird, zwischen denen eine unter Vorspannung stehende elastische Zwischenlage eingebracht ist, durch welche das Bahnmaterial unter Entspannung der Zwischenlage verkürzt und durch Ausübung von Zugbeanspruchung wieder elastisch verlängerbar ist, bei dem zunächst das Vorderende der ersten äußeren Materialschicht (20) auf den Endabschnitt eines langgestreckten stab- oder rohrartigen Formkerns (12) aufgebracht und unter gleichzeitiger Erteilung einer Vorschubbewegung auf dem Formkern (12) in eine schlauchartig geschlossene Form um den Formkern herumgefaltet und dann während der Vorschubbewegung auf dem Formkern auf die frei liegende Oberseite der ersten äußeren Materialschicht (20) Haftkleber aufgebracht wird, worauf die schlauchartige erste Schicht dann im Zuge der weiteren Vorschubbewegung auf dem Formkern (12) durch eine Zuführeinrichtungen (14) für eine Gruppe elastischen Fäden oder Strängen hindurch geführt wird, wobei die elastischen Fäden oder Stränge in der Zuführeinrichtung auf einem den langgestreckten Formkern ringförmig umgebenden Träger in in Umfangsrichtung voneinander beabstandet angeordneten Einzelführungen (18) geführt unter Vorspannung auf die erste äußere Materialschicht (20) aufgebracht und durch den Haftkleber mit dieser verbunden werden, und der ringförmigen Träger der Einzelführungen (18) während der Vorschubbewegung der schlauchförmigen ersten Materialschicht auf dem Formkern (12) um dessen Längsachse drehangetrieben wird, so dass die Fäden oder Stränge aufgrund der überlagerten Vorschubbewegung der ersten Materialschicht (20) und der Drehung des Trägers schräg verlaufend und im Wesentlichen parallel zueinander ausgerichteten auf der schlauchförmigen ersten Materialschicht (20) aufgebracht werden und diese jeweils nach Art einer Schlaucharmierung umgeben, und auf die mit den aufgebrachten Gruppen von elastischen Fäden oder Strängen belegte schlauchartige erste Materialschicht (20) dann die zweite Materialschicht (32) aufgebracht und während der weiteren Vorschubbewegung auf dem Formkern (12) schlauchförmig um diese herumgefaltet und durch Haftverbindung mit der ersten Materialschicht (20) und den aufgebrachten elastisch Fäden bzw. Strängen verbunden wird, worauf das so erzeugte schlauchförmige Bahnmaterial in Längsrichtung aufgeschnitten und zu dem im Wesentlichen ebenflächigen Bahnmaterial aufgefaltet wird,
**dadurch gekennzeichnet,**
**dass** der die Gruppe von elastischen Fäden oder Strängen auf die erste Materialschicht aufbringenden Zuführvorrichtung (14) eine in Vorschubrichtung nachgeschaltete, weitere, in entgegengesetztem Drehsinn angetriebene, zweite Zuführvorrichtung (16) vorgesehen wird, mittels welcher jeweils eine weitere Gruppe von im Wesentlichen paralell zueinander ausgerichteten gegensinnig schräg zu der ersten Gruppe verlaufenden elastischen Fäden oder Strängen auf der schlauchartigen ersten Materialschicht (20) aufgebracht und haftend mit dieser verbunden wird, und
**dass** die elastischen Fäden oder Stränge beider Gruppen während der Aufbringung auf der schlauchförmigen ersten Materialschicht (20) in gegenüber ihrem ungespannten Zustand durch eine der gewünschten elastischen Dehnung des fertigen Bahnmaterials entsprechende Vorspannung auf ein vergrößerte Länge gereckt mit der schlauchförmigen ersten Materialschicht (20) verbunden und dann die zweite Materialschicht (32) in noch gerecktem Zustand der elastischen Fäden oder Stränge auf die erste Materialschicht aufgebracht und haftend mit ihr verbunden wird.

2. Verfahren nach Anspruch 1, **dadurch gekennzeichnet, dass** der Haftkleber vor und/oder nach dem Aufbringen der elastischen Fäden oder Stränge auf der freien Oberfläche der ersten Materialschicht (20) während deren Vorschubbewegung auf dem Formkern (12) aufgebracht wird.

3. Verfahren nach Anspruch 1 oder 2, **dadurch gekennzeichnet, dass** als zweite Materialschicht (32) eine Schicht aus textilem Material verwendet wird.

4. Verfahren nach Anspruch 1 oder 2, **dadurch gekennzeichnet, dass** als.zweite Materialschicht (32) eine Folie verwendet wird.

5. Verfahren nach Anspruch 4, **dadurch gekennzeichnet, dass** eine Folie aus einem gas- oder wasserdampfdurchlässigen, gegen Flüssigkeiten jedoch undurchlässigen Folienmaterial verwendet wird.

6. Verfahren nach Anspruch 1 oder 2, **dadurch gekennzeichnet, dass** auf der mit aufgebrachten Gruppen von elastischen Fäden oder Strängen belegten ersten Materialschicht (20) als zweite Materialschicht (32) eine Lage eines in fliessfähigem Zustand befindlichen Kunststoffs aufgebracht wird, der nachfolgend in einen biegeweichen, folien- oder vliesartigen Zustand übergeführt wird.

7. Verfahren nach Anspruch 6, **dadurch gekennzeichnet, dass** als Kunststoff ein durch Erwärmung in fliessfähigen Zustand gebrachtes thermoplastisches Kunststoffmaterial verwendet wird, welches nach der Aufbringung auf der mit aufgebrachten Gruppen von elastischen Fäden oder Strängen belegten ersten Materialschicht (20) durch Abkühlung in den folien- oder vliesartigen Zustand übergeführt wird.

8. Verfahren nach Anspruch 6, **dadurch gekennzeichnet, dass** als Kunststoff ein durch Verdunstung oder Verdampfung eines Lösungsmittelanteils verfestigbares oder ein durch Vermischen von wenigstens zwei flussigen Komponenten durch Vernetzung verfestigbahres Kunststoffmaterial verwendet wird.

9. Verfahren nach einem der Ansprüche 1 bis 8, **dadurch gekennzeichnet, dass** ein mit wenigstens zwei in Umfangsrichtung zueinander versetzten, in Längsrichtung verlaufenden schlitz- oder rinnenförmigen Vertiefungen (36) versehener Formkern (12) verwendet wird, und dass beim Aufbringen der ersten Materialschicht (20) auf den Formkern (12) über den schlitz- bzw. rinnenfförmigen Vertiefungen aufgebrachte streifenförmige Bereiche der Materialschicht in die Vertiefungen eingeformt und während der Vorschubbewegung in den Vertiefungen gehalten werden.

10. Verfahren nach einem der Ansprüche 1 bis 9, **dadurch gekennzeichnet, dass** vor oder während der Aufbringung der zweiten Materialschicht (32) auf der mit Gruppen von elastischen Fäden oder Strängen versehenen ersten Materialschicht (20) ein pulver-, flocken- oder faserförmiger Füll- oder Funktionsstoff aufgebracht wird.

## Claims

1. A method for continuous production of a flexible, air-permeable web material with two outer material layers (20; 32) at least one of which is made from a textile material, between which layers a prestressed, elastic intermediate layer is inserted, where the web material is shortened through the release of tension in the intermediate layer, and can again be extended elastically through exertion of tensile stress, where initially, the front end of the first outer layer (20) of then textile material is applied onto the end section of a longitudinally extended rod-like or pipe-like mould core (12), and simultaneously with the initiation of a feed motion on the mould core, it is folded around the mould core in a tubular closed form, and then during the feed motion on the mould core, a pressure-sensitive adhesive is applied on the free top surface of the first outer layer (20), where upon the tubular first layer, in the course of the further feed motion on the mould core (12), is guided through a feed mechanism (14) for a group of elastic threads or strands under selectable elastic prestressing, where the elastic threads or strands are guided in the feed mechanism on a ring-shaped support surrounding the longitudinally extended mould core in individual guides arranged offset from each other in the circumferential direction, and applied prestessed onto the first outer layer, and are bound with it by the pressure-sensitive adhesive, where the ring-shaped support of the individual guides (18) during the feed motion of the tubular first layer on the mould (12) core is driven rotationally about the longitudinal axis of the mould core, so that the threads or strands are applied on the tubular first layer, because of the overlaid feed motion of the first layer and the rotation of the supports running obliquely and oriented essentially parallel to each other and surrounding the tubular first layer in each case, in the form of a tubular reinforcement, and then the second material layer (32) is applied onto the first tubular material layer (20), overlaid with the applied groups of elastic threads or strands, and during the further feed motion is folded tubularly around the mould core (12), and is bonded through adhesion to the first material layer and the applied elastic threads or strands, whereupon the so created tube-like web material is sliced open in the longitudinal direction and unfolded into an essentially plane-surface web material,
**characterised in that**
further to the first feed mechanism (14) applying the group of elastic threads or strands to the material layer (20) downstream in the feed direction hrough a further feed mechanism (16) driven in the opposite direction of rotation, in each case a further group of elastic threads or strands oriented essentially parallel to each other, running diagonally in the opposite direction to the first group, are applied to the applied group of elastic threads or strands onto the tubular layer and adhesively bonded with it, and
that the elastic threads or strands of both groups during the application onto and the adhesive bonding are bound with the tubular first material layer (20), where said threads or strands are lengthened, in contrast to their unstressed state, through a prestressing corresponding to the desired elastic elongation of the finished web material, whereupon the second material layer (32) in still lengthened state of the elastic threads or strands is applied to and adhesively bonded with the first material layer.

2. A method according to claim 1, **characterised in that** the pressure-sensitive adhesive is applied before and/or after the application of the elastic threads or strands onto the free top surface of the first material layer (20) during its feed motion on the mould core (12).

3. A method according to claim 1 or 2, **characterised in that** a textile material is used as the second material layer (32).

4. A method according to claim 1 or 2, **characterised in that** a film is used as the second material layer (32).

5. A method according to claim 4, **characterised in that** a film of a film material permeable to gas or water vapor, but impermeable to fluids is used.

6. A method according to claim 1 or 2, **characterised in that** onto the first material layer (20), overlaid with the applied groups of elastic threads or strands, as a second material layer (32) a layer of a plastic is applied in a free-flowing state, which subsequently transforms into a flexible film or mat-like state.

7. A method according to claim 6, **characterised in that** a thermoplastic plastic material transformed into a free-flowing state through heating is used as a plastic, which, after application onto the first material layer (20), which is overlaid with the applied groups of elastic threads or strands, is transformed into a film-like or mat-like state through cooling.

8. A method according to claim 6, **characterised in that** a plastic material capable of hardening through evaporation or vaporisation of a solvent, or a plastic material, of a mixture of at least two fluid components, capable of hardening through cross-linking is used as a plastic.

9. A method according to one of claims 1 through 8, **characterised in that** a mould core, provided with at least two slit-shaped or trough-shaped grooves (36) running in the longitudinal direction, offset from each other in the circumferential direction, is used, and that during application of the first material layer (20) onto the mould core over the slit-shaped or trough-shaped grooves strip-like areas of the material layer are then moulded into the grooves and are held in the grooves during the feed motion.

10. A method according to one of claims 1 through 9, **characterised in that** before or during the application of the second material layer (32) onto the first material layer (20), which is provided with the groups of elastic threads or strands, a powder, flake, or fibre fill material or functional material is applied.

## Revendications

1. Procédé pour fabriquer en continu un matériau en bande souple en flexion et perméable à l'air comprenant deux couches (20 ; 32) extérieures, dont au moins l'une est formée par un matériau textile, entre lesquelles une couche intermédiaire élastique et mise sous pré-tension est introduite, par laquelle le matériau en bande est raccourci avec la détente de la couche intermédiaire et peut être allongé de nouveau élastiquement par l'exercice d'une contrainte de traction, dans lequel, pour commencer, l'extrémité avant de la première couche de matériau (20) extérieure est appliquée sur le tronçon d'extrémité d'un noyau de moule (12) étiré en longueur, en forme de barre ou de tuyau, et est pliée tout autour du noyau de moule par l'attribution simultanée d'un mouvement d'avancement sur le noyau de moule (12) dans un moule fermé à la façon d'un flexible et de la colle adhésive est appliquée ensuite pendant le mouvement d'avancement sur le noyau de moule sur la face supérieure dégagée de la première couche de matériau (20) extérieure, après quoi la première couche de type flexible est guidée ensuite au cours du mouvement d'avancement ultérieur sur le noyau de moule (12) à travers un dispositif d'arrivée (14) pour un groupe de fils ou de boyaux élastiques, les fils ou boyaux élastiques étant appliqués dans le dispositif d'arrivée sur un support entourant à la façon d'un anneau le noyau de moule étiré en longueur, guidés dans des guides individuels (18) disposés à distance les uns des autres dans le sens périphérique, sous pré-tension sur la première couche de matériau (20) extérieure et étant reliés par la colle adhésive à cette couche, et le support annulaire des guides individuels (18) étant entraîné en rotation pendant le mouvement d'avancement de la première couche de matériau en forme de flexible sur le noyau de moule (12) autour de son axe longitudinal, de sorte que les fils ou boyaux sont appliqués, du fait du mouvement d'avancement superposé de la première couche de matériau (20) et de la rotation du support, agencés en biais et orientés sensiblement parallèlement entre eux, sur la première couche de matériau (20) en forme de flexible, et entourent cette couche respectivement à la façon d'une armature de flexible, et la seconde couche de matériau (32) est appliquée ensuite sur la première couche de matériau (20) de type flexible, occupée avec les groupes appliqués de fils ou de boyaux élastiques, est pliée en forme de flexible autour de cette couche pendant le mouvement d'avancement ultérieur sur le noyau de moule (12) et est reliée par liaison adhésive à la première couche de matériau (20) et aux fils et boyaux élastiques appliqués, après quoi le matériau en bande en forme de flexible ainsi généré est entaillé dans le sens de la longueur et plié par le dessus pour former le matériau en bande sensiblement plan,
**caractérisé en ce que :**
il est prévu un second dispositif d'arrivée (16) entraîné dans le sens de rotation opposé, placé en aval dans le sens d'avancement du dispositif d'arrivée (14) appliquant le groupe de fils ou de boyaux élastiques sur la première couche de matériau, second dispositif au moyen duquel à chaque fois un autre groupe de fils ou de boyaux élastiques, orientés sensiblement parallèlement entre eux et agencés dans le sens opposé en biais par rapport au premier groupe, est appliqué sur la première couche de matériau (20) de type flexible et relié de façon adhésive à celle-ci, et
**en ce que** les fils ou boyaux élastiques des deux groupes sont reliés à la première couche de matériau (20) en forme de flexible pendant l'application sur la première couche de matériau (20) en forme de flexible, étirés par rapport à leur état non tendu par une pré-tension, correspondant à l'extension élastique souhaitée du matériau en bande achevé, jusqu'à une longueur accrue et la seconde couche de matériau (32) est appliquée ensuite dans l'état encore étiré des fils ou des boyaux élastiques sur la première couche de matériau et reliée de façon adhésive avec cette couche.

2. Procédé selon la revendication 1, **caractérisé en ce que** la colle adhésive est appliquée sur le noyau de moule (12) avant et/ou après l'application des fils ou boyaux élastiques sur la surface libre de la première couche de matériau (20) pendant son mouvement d'avancement.

3. Procédé selon la revendication 1 ou 2, **caractérisé en ce qu'**une couche à base de matériau textile est utilisée en tant que seconde couche de matériau (32).

4. Procédé selon la revendication 1 ou 2, **caractérisé en ce qu'**un film est utilisé en tant que seconde couche de matériau (32).

5. Procédé selon la revendication 4, **caractérisé en ce qu'**un film à base d'un matériau en film perméable au gaz ou à la vapeur d'eau, mais imperméable aux liquides est utilisé.

6. Procédé selon la revendication 1 ou 2, **caractérisé en ce qu'**une couche d'une matière plastique se trouvant dans l'état fluide est appliquée sur la première couche de matériau (20), occupée avec des groupes appliqués de films ou boyaux élastiques, en tant que seconde couche de matériau (32), laquelle matière plastique est transférée ensuite dans un état souple en flexion, de type film ou non-tissé.

7. Procédé selon la revendication 6, **caractérisé en ce que** l'on utilise en tant que matière plastique une matière thermoplastique amenée par réchauffement dans l'état fluide, laquelle matière est transférée, après l'application sur la première couche de matériau (20) occupée avec des groupes appliqués de fils ou de boyaux élastiques par refroidissement, dans l'état de type film ou de type non-tissé.

8. Procédé selon la revendication 6, **caractérisé en ce que** l'on utilise en tant que matière plastique un matériau plastique pouvant être consolidé par évaporation d'une fraction de solvant ou un matériau plastique pouvant être consolidé par réticulation par le mélange d'au moins deux composants liquides.

9. Procédé selon l'une quelconque des revendications 1 à 8, **caractérisé en ce qu'**un noyau de moule (12) doté d'au moins deux cavités (36) en forme de fente ou de nervure, décalées dans le sens périphérique l'une par rapport à l'autre et agencées dans le sens de la longueur, est utilisé et **en ce que**, lors de l'application de la première couche de matériau (20) sur le noyau de moule (12), des zones en forme de bande, appliquées au-dessus des cavités en forme de fente ou de nervure, de la couche de matériau sont formées dans les cavités et sont maintenues dans les cavités pendant le mouvement d'avancement.

10. Procédé selon l'une quelconque des revendications 1 à 9, **caractérisé en ce qu'**un produit de remplissage ou fonctionnel en forme de poudre, de flocon ou de fibre est appliqué avant ou pendant l'application de la seconde couche de matériau (32) sur la première couche de matériau (20) dotée de groupes de fils ou de boyaux élastiques.
